# EUROPEAN PATENT SPECIFICATION

(11) **EP 2 752 484 B1**
(45) Date of publication and mention of the grant of the patent: **10.01.2018**
(21) Application number: 11871607.5
(22) Date of filing: 06.09.2011
(51) Int. Cl.: C12N 5/0775, C12N 1/38, A61K 35/12

(54) **METHOD FOR PREPARING A BASIC CULTURE MEDIUM FOR MESENCHYMAL STEM CELLS, BASIC CULTURE MEDIUM FOR MESENCHYMAL STEM CELLS, AND CELL THERAPEUTIC AGENT CULTURED AND DIFFERENTIATED USING SAME**
VERFAHREN ZUR HERSTELLUNG EINES BASISCHEN KULTURMEDIUMS FÜR MESENCHYMALE STAMMZELLEN, BASISCHES KULTURMEDIUM FÜR MESENCHYMALE STAMMZELLEN UND
PROCÉDÉ DE PRÉPARATION DE MILIEU DE CULTURE BASIQUE ET MILIEU DE CULTURE BASIQUE POUR CELLULES SOUCHES MÉSENCHYMATEUSES, ET AGENT THÉRAPEUTIQUE CELLULAIRE MIS EN CULTURE ET DIFFÉRENCIÉ À L'AIDE DE CELUI-CI

(30) Priority: 31.08.2011 KR 20110087498
(43) Date of publication of application: 09.07.2014
(73) Proprietor: Sewon Cellontech Co., Ltd, Yeongdeungpo-gu, Seoul 150-724 (KR)
(72) Inventor: SUH, Dong-Sam, Seoul 139-924 (KR); LEE, Jun Keun, Seoul 131-220 (KR); CHANG, Dong Il, Seoul 158-748 (KR); CHOI, Min Jung, Bucheon-si Gyeonggi-do 420-859 (KR); KIM, Jang Hoon, Seoul 135-231 (KR); KIM, Ga Ram, Seoul 143-825 (KR); CHANG, Cheong Ho, Seoul 135-280 (KR)
(74) Representative: dompatent von Kreisler Selting Werner - Partnerschaft von Patent- und Rechtsanwälten mbB
(86) International application number: PCT/KR2011/006582
(87) International publication number: WO 2013/032052

(56) References cited:
- WO-A1-2007/069813
- WO-A1-2007/123363
- WO-A1-2007/149328
- KR-B1- 101 037 002
- KR-B1- 101 037 008
- US-A- 5 573 937
- RAKHI PAL ET AL: "Phenotypic and functional comparison of optimum culture conditions for upscaling of bone marrow-derived mesenchymal stem cells", JOURNAL OF TISSUE ENGINEERING AND REGENERATIVE MEDICINE, vol. 3, no. 3, 19 February 2009 (2009-02-19), pages 163-174, XP055173547, ISSN: 1932-6254, DOI: 10.1002/term.143
- KAROLINA TURNOVCOVA ET AL: "Properties and growth of human bone marrow mesenchymal stromal cells cultivated in different media", CYTOTHERAPY, vol. 11, no. 7, 10 November 2009 (2009-11-10), pages 874-885, XP055060596, ISSN: 1465-3249, DOI: 10.3109/14653240903188947
- GANG ET AL.: 'In vitro mesengenic potential of human umbilical cord blood-derived mesenchymal stem cells' BIOCHEMICAL AND BIOPHYSICAL RESEARCH COMMUNICATIONS vol. 321, 2004, pages 102 - 108, XP004521404
- HILDEBRANDT ET AL.: 'Influence of cell culture media conditions on the osteogenic differentiation of cord blood-derived mesenchymal stem cells' ANNALS OF ANATOMY vol. 191, 2009, pages 23 - 32, XP025870120

## Description

### Technical Field

The present invention relates to an advanced basic culture medium for mesenchymal stem cells. Also disclosed are a basic culture medium for mesenchymal stem cells derived from bone marrow and fat; a method for creating a basic culture medium for mesenchymal stem cells, the method being capable of, during the *in vitro* culturing procedure of mesenchymal stem cells derived from bone marrow and fat, increasing the growth rate of mesenchymal stem cells compared with the conventional art of culturing mesenchymal stem cells using commercialized media to shorten the time from collection to mass culture, and multi-differentiating the early cultured mesenchymal stem cells into therapeutic agents of osteoblasts, chondrocytes, and adipocytes; a basic culture medium for mesenchymal stem cells; and a cell therapeutic agent cultured and differentiated using the same.

### Background Art

Stem cell technology has been recently presented as a new field for intractable disease treatment in regenerative medicine using tissue engineering. Hence, there is a rising interest in studying stem cells, and stem cells capable of forming tissues through proliferation and differentiation are recognized to solve most diseases, tissue damage, and the like.

Stem cells have auto-replication capability in an undifferentiated state, and differentiate into specialized cells under appropriate conditions. Stem cells may be divided into embryonic stem cells and adult stem cells depending on the origin thereof. Since human embryonic stem cells are obtained from an embryo with the possibility of developing into a human being, they raise bioethical problems in spite of excellent cell proliferation and differentiation capabilities thereof. The adult stem cells have limited differentiation capacity when compared with the embryonic stem cells, but are less problematic in view of bioethics since the cells pre-existing in various human organs are collected from bone marrow, blood, brain, skin, and the like, thereby producing stem cells.

Mesenchymal stem cells were first isolated from adult bone marrow (Y. Jiang et al., Nature, 418:41, 2002), and then also found in the skin, blood vessel, muscle, and brain tissues, like in the bone marrow (J.G. Toma et al., Nat. Cell Biol., 3:778, 2001; M. Sampaloesi et al., Science, 301:487, 2003; Y. Jiang et al., Hematol., 30:896, 2002). In addition, adipose-derived mesenchymal stem cells having differentiation capacity, like in the bone marrow, were recently found in the adipose tissue (B. Cousin et al,. BBRC., 301:1016, 2003; A. Miranville et al., Circulation, 110:349, 2004; S. Bronthos et al., J. Cell Physiol., 189:54, 2001; M.J. Seo et al., BBRC., 328:258, 2005).

Techniques of isolating mesenchymal stem cells from human bone marrow and techniques of isolating mesenchymal stem cells from adipose tissues are respectively disclosed in literature by Pittenger, et al. (Science 284: 143, 1997) and van, et al. (J. Clin. Invest., 58: 699, 1976). In these literatures, α-MEM or DMEM and 10-20% fetal bovine serum were used for cell culture.

However, mesenchymal stem cells are very rarely present in adult tissues, such as bone marrow and adipose tissue, and such cells have a low growth rate and are difficult to maintain for a long time in an undifferentiated state, and therefore, such cells are hard to preserve through *in vitro* proliferation and culture without specifically screened media.

The mammal cell culture medium is composed of about 50 components, which may be largely classified into ones used for cellular biosynthesis, ones used for biological energy metabolism, and ones serving as catalysts for several metabolic actions or for regulating intracellular physiological phenomena. That is, the media used for cell culture is composed of isotonic solution components, buffer solution components, nutritional components including amino acids as energy sources, vitamins, and inorganic salts, and other various kinds of supplements.

In addition, the supply of approximately 5-20% of serum depending on cellular characteristics provides various hormones, growth factors, fats, and vitamins, which are suitable for cell proliferation, inhibits the activity of protease, and serves as a buffer for pH adjustment, thereby promoting the growth and activity of mammal cells. As for the components constituting the media for culturing mammal cells, the concentrations and composition of the components vary depending on the type of each medium. Through this method, Morgan, et al. created M119 media based on the body fluid composition in 1950's, and cultured fetal cells (primary chick) (Morgan, et al., 1950).

Culture media may be largely classified into media that are more simple and commonly used for cell culture, such as minimal essential medium (MEM; Eagle, 1955), Rosewel Park Memorial Institute (RPMI)-1640 (Moore *et al.,* 1967), and Dulbecco's modification of Eagle's medium (DMEM; Dulbecoo, *et al.,* 1959), and media that are more complex and have several enriched components, such as Iscove's modification of DMEM (IMDM), Ham's F-12 (Ham, 1965), and Connaught Medical Research Laboratories (CMRL)-1666.

The growth curve of mammal cells has a lag phase of normally 2-3 days, and the concentration of living cells is rapidly reduced from the end of the lag phase due to the accumulation of ammonium, which is a glutamine metabolite, and lactic acid, which is a glucose metabolite. Glucose and glutamine are used as a main carbon source and an energy source in main metabolic pathways of mammal cells. Glucose is metabolized into pyruvic acid through glycolysis in mammal cells. In addition, pentose is prepared through a pentose phosphate pathway, resulting in nucleic acid synthesis. The pyruvic acid generated through glycolysis may be decomposed into carbon dioxide and water or may become lactic acid through the TCA cycle, and also may become fatty acid. Glutamine is the unique out of the carbon sources used in the mammal cell metabolism. In the metabolic process, some glutamine becomes glutamate, which then enters the TCA cycle to form a carbon skeleton for synthesizing other amino acids. The main excrements of mammal cells are lactic acid and ammonia, and the excretion of alanine is also important. Lactate and ammonia change the intracellular pH and the lysosomal pH, and thus act as toxins on cells. Since physiological mechanisms and nutritional requirements vary depending on the type of cultured cells as such, the type of medium used in the cell culture needs to be also varied depending on the characteristics of the medium.

Therefore, in order to *in vitro* proliferate and culture undifferentiated mesenchymal stem cells derived from adult tissues, such as human bone marrow and adipose tissue, the composition of the medium suitable for growth conditions of the undifferentiated mesenchymal stem cells will be different.

Conventionally, many studies about culture media for *in vitro* proliferating and culturing undifferentiated mesenchymal stem cells have been ongoing as follows.

Patent Document 1 (Method for Mass-Producing Growth Factor Using Mesenchymal Stem Cells) discloses a serum-free medium which is supplemented with Ham's F-12 on the basis of DMEM to promote the differentiation of growth factors from mesenchymal stem cells, thereby synthesizing significantly large amounts of human growth factors. However, this is a basic medium in which DMEM is mixed with Ham's F-12, for mass-producing basic fibroblast growth factor, vascular endothelial growth factor, or human transforming growth factor-beta from mesenchymal stem cells rather than proliferating mesenchymal stem cells.

Patent Document 2 (Medium Composition Necessary for *In Vitro* Proliferation of Mesenchymal Stem Cells Derived from Umbilical Cord Blood, Containing Soy Protein Hydrolysate) relates to the technique of adding a soy protein hydrolysate as a component of the stem cell culture medium to a low-glucose DMEM medium containing fetal bovine serum, in order to reduce the amount of the fetal bovine serum.

Patent Document 3 (Method for Preparing Dermal Papilla Tissue Using Mesenchymal Stem Cells) and Patent Document 4 (Method for Preparing Dermal Papilla Tissue Using Mesenchymal Stem Cells) relate to the technique of culturing mesenchymal stem cells in DMEM, DMEM/F-12, F-12, McCoy's 5A, RPMI 1640, Williams' medium E, or Iscove's modified Dulbecco's modification (IMDM), and then inducing the differentiation into dermal papilla tissues, and describe a medium obtained by adding hydrocortisone, insulin, transferrin, and sodium selenite to a commercialized basic cell culture medium.

Patent Document 5 (Mesenchymal Stem Cell Culture Medium and Method for Culturing Mesenchymal Stem Cells Using Same) relates to the technique of culturing mesenchymal stem cells by using a commercialized medium supplemented with a nutrient mixture as a base and further adding insulin, hydrocortisone, EGF, LIF, GM-CSF, and the like, which are growth factors of mesenchymal stem cells, and describes a culture technique of mixing a nutrient mixture with the commercialized culture medium.

Patent Document 6 (Multipotent Stem Cells Derived from Human Adipose Tissue and Cellular Therapeutic Agents Comprising Same) relates to the technique of proliferating mesenchymal stem cells by using DMEM as a base and adding Keratinocyte-SFM supplemented with NAC, rEGF, BPE, insulin, and the like, wherein the basic cell culture medium is DMEM.

Patent Document 7 (Composition for Preventing or Treating Cancer, Containing Adult Stem Cell Culture or Fraction Thereof) and Patent Document 8 (Isolated Pluripotent Adult Stem Cells and Methods for Isolating and Culturing Same) relate to the technique of culturing mesenchymal stem cells using DMEM/Ham's F-12 mixture medium, DMEM, and DMEM/F-12.

As indicated above, the conventional techniques are limited to the culturing by adding additives, such as growth factors, to the commercialized medium as a base.

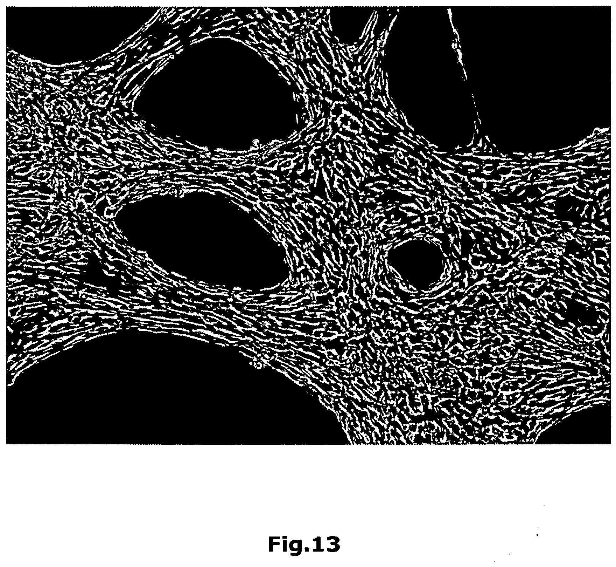

Meanwhile, Patent Document 9 (Chondrocyte-Specific Culture Method for Early Culture of Chondrocytes) filed and registered by the present applicant relates to the development of the culture medium in which additives are not added to the conventional commercialized medium. In Patent Document 9, mesenchymal stem cells isolated from adult tissues, such as bone marrow and adipose tissue, were cultured by using the advanced basic medium-chondrocyte (ABM-C). However, morphologic abnormality and the loss of adhesiveness occurred during the cell culture as shown in FIG. 13, and thus the mesenchymal stem cells floated on the cell culture medium, so mesenchymal stem cells that adhere and proliferate *in vitro* no longer proliferated.

Further documents that may be mentioned are: WO 2007/149328 A1; WO 2007/123363 A1;, WO 2007/069813 A1; Rakhi Pal et al. "Phenotypic and functional comparison of optimum culture conditions for upscaling of bone marrow-derived mesenchymal stem cells"; Journal of Tissue Engineering and Regenerative Medicine, vol. 3, no. 3, 1 March 20009 (2009-03-01), pages 163-174 and Karolina Turnovcova et al., "Properties and growth of human bone marrow mesenchymal stromal cells cultivated in different media", Cytotherapy, vol. 11, no. 7, 10 November 2009 (2009-11-10), pages 874-885.

### [Prior Art Documents]

### [Patent Documents]

(Patent Document 1) Korean Patent Registration No. 10-0899329
(Patent Document 2) Korean Patent Publication No. 10-2009-0090850
(Patent Document 3) Korean Patent Registration No. 10-1022032
(Patent Document 4) Korean Patent Publication No. 10-2010-0110905
(Patent Document 5) Korean Patent Registration No. 10-0908481
(Patent Document 6) Korean Patent Registration No. 10-0679642
(Patent Document 7) Korean Patent Publication No. 10-2009-0121541
(Patent Document 8) Korean Patent Publication No. 10-2006-0010847
(Patent Document 9) Korean Patent Registration No. 10-1037002

### Detailed Description of the Invention

### Technical Problem

Therefore, the present invention has been made in view of the above-mentioned problems, and an aspect of the present invention is to provide a basic culture medium for mesenchymal stem cells, capable of massively proliferating and culturing undifferentiated mesenchymal stem cells derived from adult tissues, such as human bone marrow and adipose tissues, at a high growth rate.

Another aspect of the present disclosure is to provide a cell therapeutic agent containing undifferentiated mesenchymal stem cells, wherein the cell therapeutic agent *in vitro* differentiates the undifferentiated mesenchymal stem cells, derived from adult cells, into osteoblasts, chondrocytes, and adipocytes, by using the basic culture medium for mesenchymal stem cells.

### Technical Solution

In accordance with the present invention, there is provided an advanced basic culture medium for mesenchymal stem cells comprising the components and their respective amounts in mg/L as given in the following table:

| component | amount [mg/l] |
|---|---|
| Glycine | 30 |
| L-alanine | 9 |
| L-arginine monohydrochloride | 211 |
| L-asparagine anhydrous | 50 |
| L-aspartic acid | 20 |
| L-cystine dihydrochloride | 62.6 |
| L-glutamic acid | 20 |
| L-glutamine | 584 |
| L-histidine monohydrochloride | 42 |
| L-hydroxy-L-proline | 20 |
| L-isoleucine | 105 |
| L-leucine | 105 |
| L-lysine monohydrochloride | 146 |
| L-methionine | 30 |
| L-phenylalanine | 66 |
| L-proline | 34.5 |
| L-serine | 42 |
| L-threonine | 95 |
| L-tryptophan | 16 |
| L-tyrosine disodium salt dihydrate | 103.79 |
| L-valine | 94 |
| Calcium chloride dihydrate | 265 |
| Cupric sulfate pentahydrate | 0.0025 |
| Ferrous sulfate heptahydrate | 0.834 |
| Magnesium sulfate anhydrous | 97.67 |
| Potassium chloride | 400 |
| Sodium chloride | 6400 |
| Sodium phosphate dibasic anhydrous | 142.04 |
| Zinc sulfate heptahydrate | 0.863 |
| Ascorbic acid phosphate | 50 |
| Choline chloride | 13.96 |
| D-biotin | 0.2 |
| D-Ca panthothenate | 4 |
| Folic acid | 4 |
| Myo-inositol | 35 |
| Nicotinamide (nicotinic acid amide) | 4 |
| p-aminobenzoic acid (PABA) | 1 |
| Pyridoxal hydrochloride | 4 |
| Riboflavin | 0.4 |
| Thiamine hydrochloride | 4 |
| Vitamine B12 | 1.36 |
| D-glucose anhydrous | 4500 |
| hypoxanthine | 4.08 |
| L-glutathione reduced | 1 |
| Linoleic acid | 0.084 |
| Phenol red sodium salt | 15.9 |
| Putrescine+2HCl | 0.161 |
| Sodium pyruvate | 110 |
| Thioctic acid | 0.21 |
| thymidine | 0.73 |

In accordance with an aspect of the present disclosure, there is provided a method for creating a basic culture medium for mesenchymal stem cells, the method including: preparing several kinds of basic culture medium composition candidates by analyzing the kind of components of commercialized culture media and then combining two or more kinds of commercialized culture media; and screening a basic culture medium composition suitable for early culture of mesenchymal stem cells by analyzing the growth rate of mesenchymal stem cells in a complete medium containing 10-20% fetal bovine serum for the basic culture medium composition candidates.

In accordance with another aspect of the present disclosure, there is provided a basic culture medium for mesenchymal stem cells, wherein an advanced basic media-mesenchymal stem cell (ABM-M) is created by comparing a basic medium in which DMEM high glucose, RPMI-1640, and Ham's F-12, as commercialized media, are mixed at a ratio of 1:1:1, with DMEM high glucose, RPMI-1640, and Ham's F-12, respectively, and using components of the DMEM high glucose as basic components, wherein overlapping components of the respective media are selected to have higher concentrations, while the overlapping components are allowed to be contained in the DMEM high glucose if the overlapping components are not contained in the DMEM high glucose, wherein components contained in only one of the respective media are selected to maintain concentrations thereof *per se,* while the components are allowed to be contained in the DMEM high glucose if the components are not contained in the DMEM high glucose, and wherein one component selected from components corresponding to the same supply source among the components of the respective media is selected to have a higher concentration if the selected component is an overlapping component of the respective media, while the selected component is allowed to be contained in the DMEM high glucose if the overlapping components are not contained in the DMEM high glucose.

In the basic culture medium for mesenchymal stem cells, the components added to the DMEM high glucose may include: L-alanine, L-asparagine anhydrous, L-aspartic acid, L-glutamic acid, L-hydroxy-L-proline, and L-Proline in view of amino acids; cupric sulfate pentahydrate, ferrous sulfate heptahydrate, sodium phosphate dibasic anhydrous, and zinc sulfate heptahydrate in view of inorganic salts; D-biotin, P-aminobenzoic acid (PABA), and vitamin B12 in view of vitamins; and hypoxanithine, L-glutathione reduced, linoleic acid, putrescine+2HCL, thioctic acid, and thymindine in view of other components.

In the basic culture medium for mesenchymal stem cells, the ABM-M may further include at least one of fetal bovine, equine, or human serum, L-glutamine, antibiotic agents, and antifungal agents.

In the basic culture medium for mesenchymal stem cells, the ABM-M may further include 10-20% fetal bovine serum and 2-4 mM L-glutamine.

In the basic culture medium for mesenchymal stem cells, the one component selected from the components corresponding to the same supply source may include: L-arginine monobydrochloride, L-aspartic acid, L-cystine dihydrochloride, and L-histidine monobydrochloride monohydrate in view of amino acids; calcium chloride dehydrate, ferrous sulfate heptahydrate, magnesium sulfate anhydrous, and sodium phosphate dibasic anhydrous in view of inorganic salts; and pyridoxal hydrochloride in view of vitamins.

In the basic culture medium for mesenchymal stem cells, the ABM-M may express CD166, CD105, CD90, CD44, CD29, CD73, and HLA-ABC positive surface markers at 80% or more, especially CD44, CD105, CD90, CD73, and CD166 positive surface markers at 95% or more, and CD14, CD31, CD34, CD45, CD80, and HLA-DR negative surface markers at 5% or less.

In accordance with still another aspect of the present disclosure, there is provided a cell therapeutic agent for treating bone defects, wherein mesenchymal stem cells are cultured in the advanced basic media-mesenchymal stem cell (ABM-M), and then again cultured and differentiated in α-MEM containing fetal bovine serum, dexamethasone, β-glycerophosphate, and ascorbic acid, leading to the differentiation into osteoblasts.

In accordance with still another aspect of the present disclosure, there is provided a cell therapeutic agent for treating ostarthritis, wherein mesenchymal stem cells are cultured in the advanced basic media-mesenchymal stem cell (ABM-M), and then again cultured and differentiated in α-MEM containing dexamethasone, ascorbic acid, sodium pyruvate, TGF-β, and BMP-2, leading to the differentiation into chondrocytes.

In accordance with still another aspect of the present disclosure, there is provided a cell therapeutic agent for forming adipocytes, wherein mesenchymal stem cells are cultured in the advanced basic media-mesenchymal stem cell (ABM-M), and then again cultured and differentiated in α-MEM containing fetal bovine serum, dexamethasone, indomethacin, and insulin, leading to the differentiation.

### Advantageous Effects

The undifferentiated mesenchymal stem cells, which are human adult stem cells, can be mass-produced and cultured at a high growth rate by the foregoing basic culture medium for mesenchymal stem cells of the present invention; the karyotype of cells can be maintained in spite of the culture and proliferation for a long period of time of one month or longer; and the mesenchymal stem cells can be used as a cell therapeutic agent through differentiation into derivatives of osteoblasts, chondrocytes, and adipocytes.

### Brief Description of the Drawings

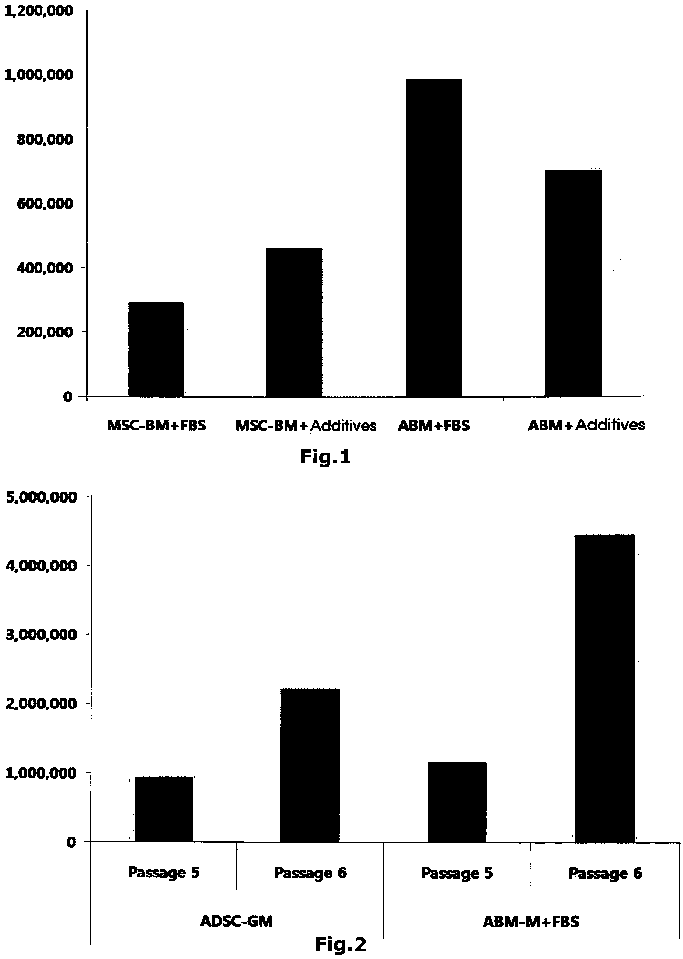

FIG. 1 shows a graph illustrating the growth of mesenchymal stem cells, after bone marrow-derived mesenchymal stem cells were inoculated at 375,000 cells in media containing MSC-BM, as a control group, plus additives and 10% fetal bovine serum, and media containing ABM-M of the present invention plus additives and 10% fetal bovine serum in T75 flasks, followed by culturing for 10 days.
FIG. 2 shows a graph illustrating the growth of mesenchymal stem cells, after adipose-derived mesenchymal stem cells are inoculated at 375,000 cells in ADSCGM as a control group and the medium containing ABM-M of the present invention plus 10% fetal bovine serum in T75 flasks, followed by culturing for 7 days, and the mesenchymal stem cells are grown and subcultured, and then inoculated at 875,000 cells in T175 flasks, followed by culturing for 7 days.

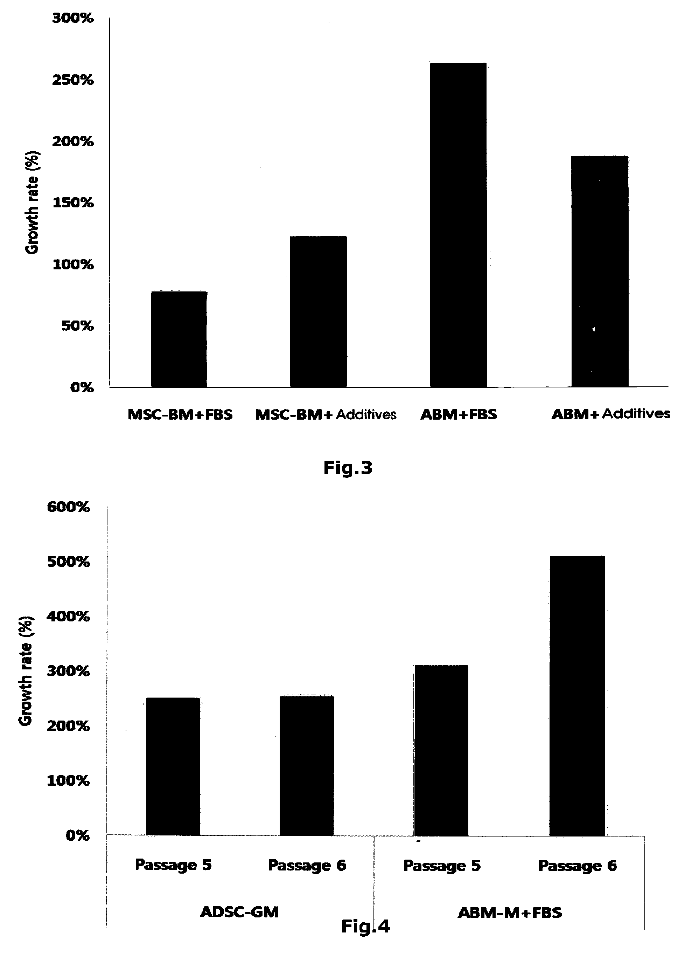

FIG. 3 shows a graph illustrating the growth rate of mesenchymal stem cell by growing bone marrow-derived mesenchymal stem cells in media containing MSC-BM, as a control group, plus additives and 10% fetal bovine serum, and media containing ABM-M of the present invention plus additives and 10% fetal bovine serum
FIG. 4 shows a graph illustrating the growth rate of mesenchymal stem cell for each passage by growing adipose-derived mesenchymal stem cells in ADSCGM as a control group and the medium containing ABM-M of the present invention plus 10% fetal bovine serum.

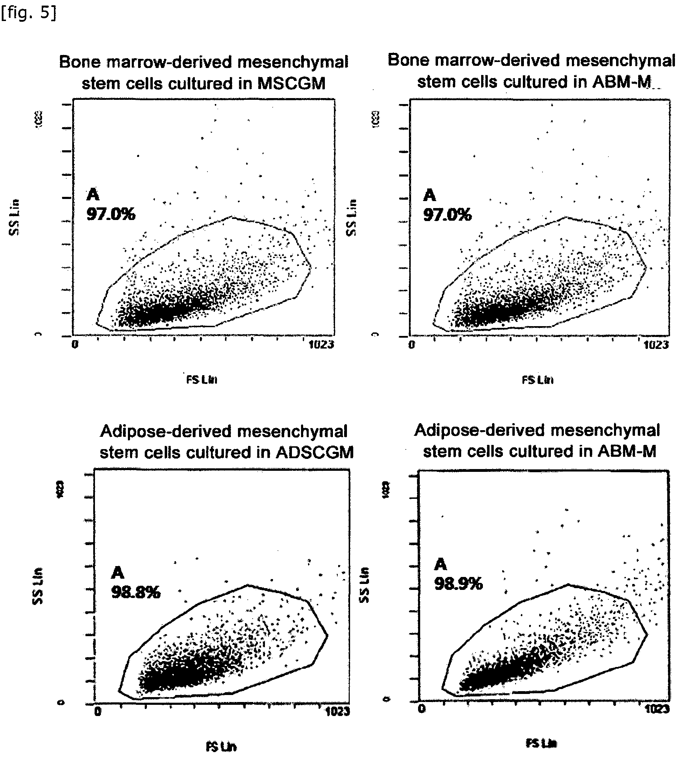

FIG. 5 shows graphs illustrating changes in the size and phenotype of cells by performing flow cytometry on bone marrow- or adipose-derived mesenchymal stem cells cultured in MSCGM or ADSCGM as a control group and bone marrow- or adipose-derived mesenchymal stem cells cultured in media containing ABM-M of the present invention plus 10% fetal bovine serum and 2 mM L-glutamine (SS; granular content within cell, FS; cell size).

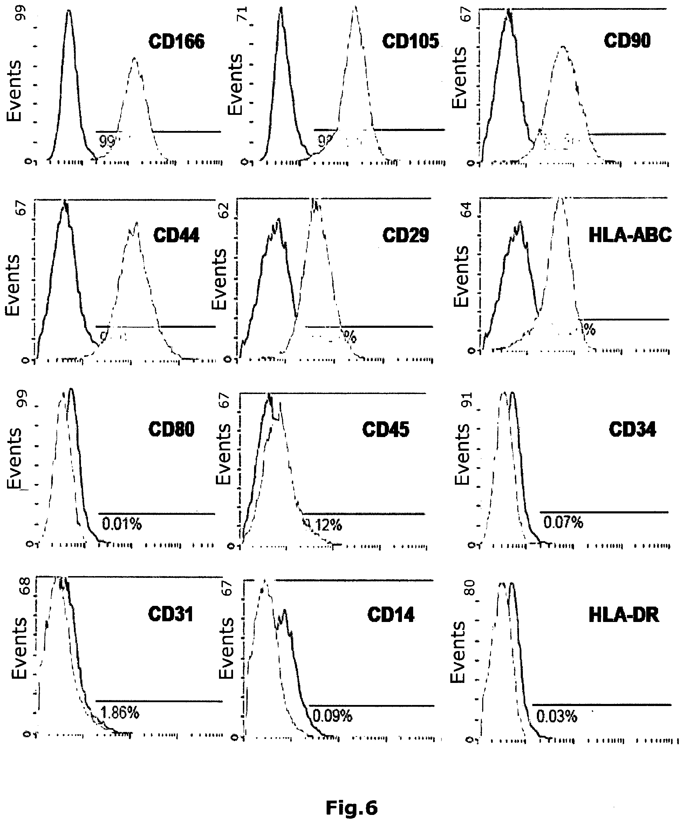

FIG. 6 shows histograms illustrating cell immunological characteristics of bone marrow-derived mesenchymal stem cells cultured in media containing ABM-M of the present invention plus 10% fetal bovine serum and 2 mM L-glutamine.

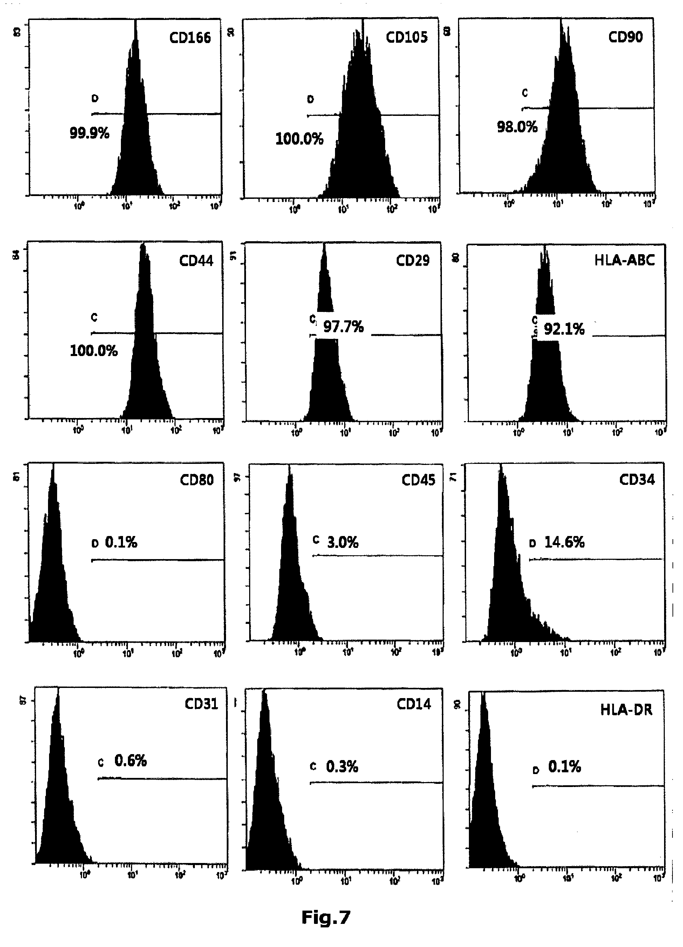

FIG. 7 shows histograms illustrating cell immunological characteristics of adipose-derived mesenchymal stem cells cultured in media containing ABM-M of the present invention plus 10% fetal bovine serum and 2 mM L-glutamine.

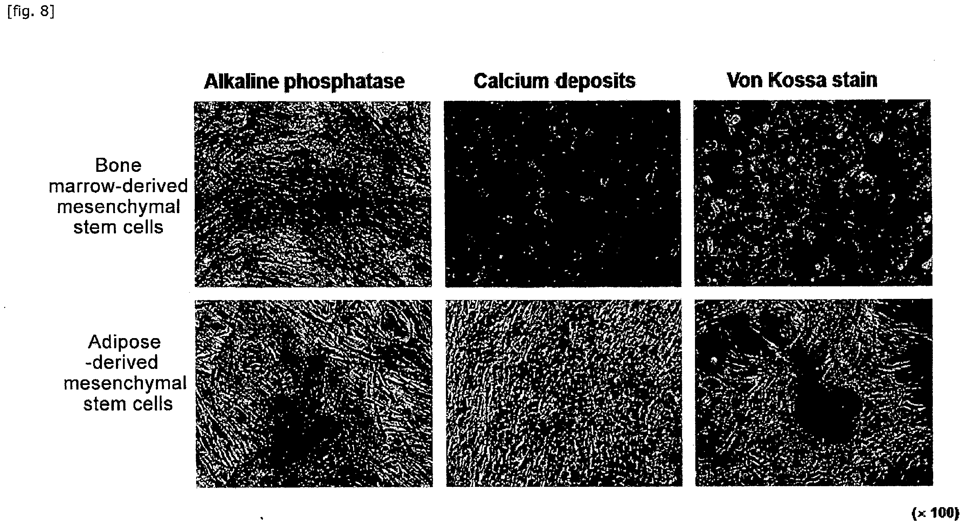

FIG. 8 shows ALPase and von Kossa staining images after bone marrow- and adipose-derived mesenchymal stem cells were cultured in media containing ABM-M of the present invention plus 10% fetal bovine serum and 2 mM L-glutamine, and then again cultured and differentiated in culture media for osteoblast differentiation (α-MEM containing 10% fetal bovine serum, 10 mM β-glycerol phosphate, 50 uM ascorbic acid, and 10⁻⁷ M dexamethasone) for 2-3 weeks.

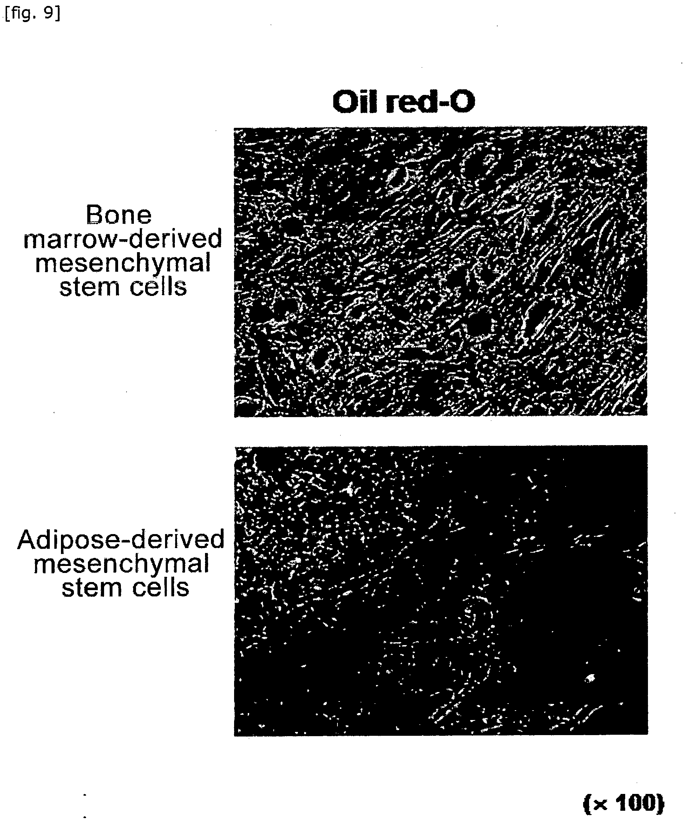

FIG. 9 shows oil Red-O staining images after bone marrow- and adipose-derived mesenchymal stem cells were cultured in media containing ABM-M of the present invention plus 10% fetal bovine serum and 2 mM L-glutamine, and then re-cultured and differentiated in media for adipocyte differentiation (α-MEM containing 10% fetal bovine serum, 10⁻⁷ M dexamethasone, 100 uM indomethacin, and 10 ug/mL insulin) for 2 weeks.

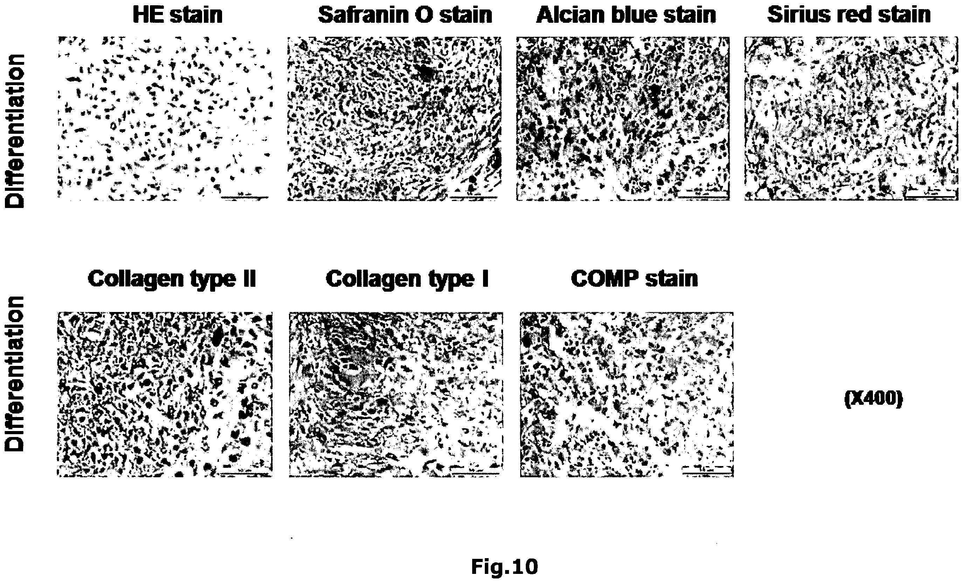

FIG. 10 shows H/E staining, safranin O staining, alcian blue staining, sirius red staining, COMP staining, and collagen type II and I staining images of 5-um serial sections of cartilage tissue through a paraffin embedding procedure, the cartilage tissue being obtained by culturing bone marrow-derived mesenchymal stem cells cultured in media containing ABM-M of the present invention plus 10% fetal bovine serum and 2 mM L-glutamine, and then, in order to induce the differentiation into chondrocytes, centrifuging approximately 300 g of about 5 10⁵ cells for 5 minutes to make a cell mass and re-culturing and differentiating the cultured mesenchymal stem cells in DMEM high glucose media containing 10⁻⁷ M dexamethasone, 50 uM ascorbic acid, 1 nM sodium pyruvate, 10 ng/mL TGF-β, and 100 ng/mL BMP-2 for 3 weeks.

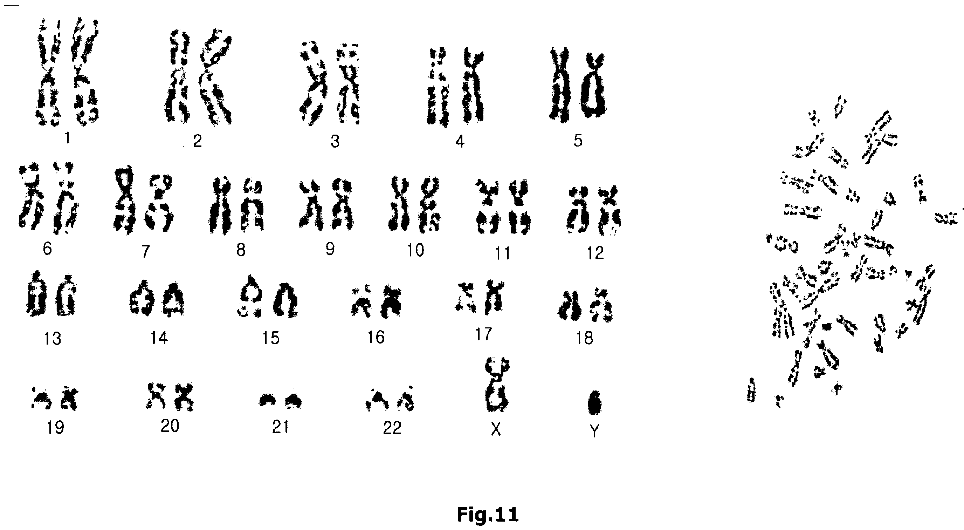

FIG. 11 shows karyotype analysis images after 10 passages in culture of bone marrow-derived mesenchymal stem cells cultured in media containing ABM-M of the present invention plus 10% fetal bovine serum and 2 mM L-glutamine.

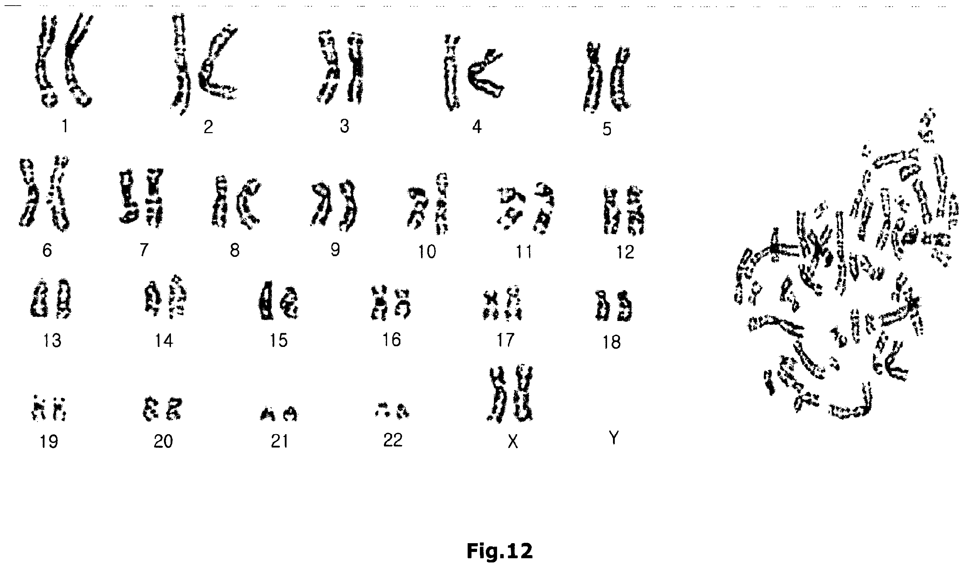

FIG. 12 shows karyotype analysis images after 10 passages in culture of adipose-derived mesenchymal stem cells cultured in media containing ABM-M of the present invention plus 10% fetal bovine serum and 2 mM L-glutamine.
FIG. 13 shows an image illustrating morphologic abnormality and loss of adhesiveness in mesenchymal stem cells while culturing mesenchymal stem cells in conventional ABM-C.

### Best Mode for Carrying Out the Invention

The present invention provides a basic culture medium for mesenchymal stem cells, and the basic culture medium for mesenchymal stem cells is created by including the steps of: preparing several kinds of basic culture medium composition candidates by analyzing the kind of components of commercialized culture media and then combining two or more kinds of commercialized culture media; and screening a basic culture medium composition suitable for early culture of mesenchymal stem cells by analyzing the growth rate of mesenchymal stem cells in a complete medium containing 10-20% fetal bovine serum for the basic culture medium composition candidates.

The thus created basic culture medium for mesenchymal stem cells of the present invention is used to analyze whether the mesenchymal stem cells cultured early at a high growth rate exhibit cell immunological characteristics, analyze differentiation capacity of whether the mesenchymal stem cells differentiate into osteoblasts, chondrocytes, and adipocytes, and analyze the cell karyotype in order to confirm whether the mesenchymal stem cells can be cultured for a long time without causing any mutations due to chromosomal abnormality, thereby verifying whether the mesenchymal stem cells can be used as a therapeutic agent for treating bone defects, treating ostarthritis, and treating adipocytes through adipose tissue formation.

Here, the cell immunological characteristics, differentiation capacity, and cell karyotype need to be analyzed to verify whether the basic culture medium for mesenchymal stem cells of the present invention can be used for a cell therapeutic agent, and it is preferable to perform verification through experiments in the following order. The verification is performed by: a step of analyzing cell immunological cell characteristics of mesenchymal stem cells cultured in the screened medium composition; a step of analyzing the differentiation capacity of the mesenchymal stem cells cultured in the screened medium composition; and a step of analyzing the cell karyotype to verify whether the mesenchymal stem cells cultured in the screened medium composition can be cultured for a long time.

Hereinafter, the present invention will be described in detail.

An advanced basic media-mesenchymal stem cell (ABM-M), which is the basic culture medium for mesenchymal stem cells of the present invention, is created by preparing a basic medium in which DMEM high glucose, RPMI-1640, and Ham's F-12, which are commercialized culture media, are mixed at a ratio of 1:1:1, and using the components of the DMEM high glucose as basic components. Here, the basic medium is compared with the respective media, DMEM high glucose, RPMI-1640, and Ham's F-12. Herein, the overlapping component of the respective media is selected to have a higher concentration, while the overlapping component is allowed to be contained in the DMEM high glucose through an addition thereof if the overlapping component is not contained in the DMEM high glucose (① in note columns of table 1); the component contained in only one of the respective media is selected to maintain the concentration thereof *per se,* while the component is allowed to be contained in the DMEM high glucose medium through an addition thereof if the component is not contained in the DMEM high glucose (② in note columns of table 1); and one component selected from component corresponding to the same supply source among the components of the respective media is selected to have a higher concentration if the selected component is an overlapping component of the respective media, while the selected component is allowed to be contained in the DMEM high glucose media through an addition thereof if the selected component is not contained in the DMEM high glucose (③ in note columns of table 1). Hence, the medium is created as shown in table 1.

**[Table 1]**

| Compositions of commercialized media and ABM-M | | | | | | |
|---|---|---|---|---|---|---|
| **Components (mg/L)** | | **DMEM** | **RPMI** | **F-12** | **ABM-M** | **NOTE** |
| ***Amino Acids*** | | | | | | |
| | **Glycine** | **30** | **10** | **7.51** | **30** | **①** |
| | **L-Alanine** | | | **9** | **9** | **②, contained** |
| | **L-Arginine Free Base** | | **200** | | | **③** |
| | **L-Arginine Monohydrochloride** | **84** | | **211** | **211** | |
| | **L-Asparagine Anhydrous** | | **50** | | **50** | **③, contained** |
| | **L-Asparagine Monohydrate** | | | **15.01** | | |
| | **L-Aspartic acid** | | **20** | **13.3** | **20** | **①, contained** |
| | **L-Cysteine Monohydrochloride Monohydrate** | | | **35** | | **③** |
| | **L-Cyctine Dihydrochloride** | **62.6** | **65.2** | | **62.6** | |
| | **L-Glutaminc Acid** | | **20** | **14.7** | **20** | **①, contained** |
| | **L-Glutamine** | **584** | **300** | **146** | **584** | **①** |
| | **L-Histidine** | | **151** | | | **③** |
| | **L-Histidine Monohydrochloride** | **42** | | **20.96** | **42** | |
| | **L-Hydroxy-L-Proline** | | **20** | | **20** | **②,** 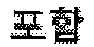 |
| | **L-Isoleucine** | **105** | **50** | **3.94** | **105** | **①** |
| | **L-Leucine** | **105** | **50** | **13.1** | **1050** | **①** |
| | **L-Lysine Monohydrachloride** | **146** | **40** | **36.5** | **146** | **①** |
| | **L-Methionine** | **30** | **15** | **4.48** | **30** | **①** |
| | **L-Phenylalanine** | **66** | **15** | **4.96** | **66** | **①** |
| | **L-Proline** | | **20** | **34.5** | **34.5** | **①, contained** |
| | **L-Serine** | **42** | **30** | **10.5** | **42** | **①** |
| | **L-Threonine** | **95** | **20** | **11.9** | **95** | **①** |
| | **L-Tryptophan** | **16** | **5** | **2.04** | **16** | **①** |
| | **L-Tyrosine Disodium Salt Dihydrate** | **103.79** | **28.83** | **7.78** | **103.79** | **①** |
| | **L-Valine** | **94** | **20** | **11.7** | **94** | **①** |

| ***Inorganic Salts*** | | | | | | |
|---|---|---|---|---|---|---|
| | **Calcium Chloride dihydrate** | **265** | | **44.1** | **265** | **③** |
| | **Calcium Nitrate Tetrahydrate** | | **100** | | | |
| | **Cuprir Sulfate Pentahydrate** | | | **0.0025** | **0.0025** | **②, contained** |
| | **Ferric Nitrate Nonahydrate** | **0.1** | | | | **③, contained** |
| | **(Ferrous Sulfate Heptahydrate** | | | **0.834** | **0.834** | |
| | **Magnesium Chloride Hexahydrate** | | | **123** | | **③** |
| | **Magnesium Sulfate Anhydrous** | **97.67** | **48.84** | | **97.67** | |
| | **Potassium Chloride** | **400** | **400** | **224** | **400** | **①** |
| | **Sodium Chloride** | **6.400** | **5,300** | **7,599** | **6,400** | **①** |
| | **Sodium Phosphate Dibasic Anhydrous** | | **800** | **142.04** | **142.04** | **③, contained** |
| | **Sodium Phosphate Monobasic Anhydrous** | **109** | | | | |
| | **Zinc Sulfate Heptahydrate** | | | **0.863** | **0.863** | **②, contained** |

| **Components (mg/L)** | | **DMEM** | **RPMI** | **F-12** | **ABM-M** | **NOTE** |
|---|---|---|---|---|---|---|
| ***Vitamins*** | | | | | | |
| | **Ascorbic Acid Phosphate** | **50** | **50** | **500** | **50** | **①** |
| | **Choline Chloride** | **4** | **3** | **13.96** | **13.96** | **①** |
| | **D-Biotin** | | **0.2** | **0.0073** | **0.2** | **①,Contained** |
| | **D-Ca Pantothenate** | **4** | **0.25** | **0.48** | **4** | **①** |
| | **Folic Acid** | **4** | **1** | **1.32** | **4** | **①** |
| | **(Myo-Inositol** | **7.2** | **35** | **18** | **35** | **①** |
| | **Nicotinamide (Nicotinic acid amide)** | **4** | **1** | **0.037** | **4** | **①** |
| | **P-Aminobenzoic Acid (PABA)** | | **1** | | **1** | **②, Contained** |
| | **Pyridoxal Hydrochloride** | **4** | | | **4** | **③** |
| | **Pyridoxine Hydrochloride** | | **1** | **0.062** | | |
| | **Riboflavin** | **0.4** | **0.2** | **0.038** | **0.4** | **①** |
| | **Thiamine Hydrochloride** | **4** | **1** | **0.34** | **4** | **①** |
| | **Vitamine B12** | | **0.005** | **1.36** | **1.36** | **①, Contained** |

| ***Other Components*** | | | | | | |
|---|---|---|---|---|---|---|
| | **D-Glucose Anhydrous** | **4,500** | **2,000** | **1,802** | **4,500** | **①** |
| | **Hypoxanthine** | | | **4.08** | **4.08** | **②,Contained** |
| | **L-Glutathione Reduced** | | 1 | | 1 | **②,Contained** |
| | **Linoieic acid** | | | **0.084** | **0.084** | **②,Contained** |
| | **(Phenol Red Sodium Salt** | **15.9** | **5.3** | **1.3** | **15.9** | **①** |
| | **Putrescine+2HCL** | | | **0.161** | **0.161** | **②,Contained** |
| | **Sodium Pyruvate** | **110** | | **110** | **110** | **①** |
| | **Thioctic Acid** | | | **0.21** | **0.21** | **②,Contained** |
| | **Thymidine** | | | **0.73** | **0.73** | **②,Contained** |

As shown in note columns of table 1, the components that are absent in the DMEM high glucose, which is the base of ABM-M of the present invention, and thus are allowed to be contained in the DMEM high glucose through an addition are L-Alanine (9-18 mg/L), L-Asparagine Anhydrous (1-50 mg/L), L-Aspartic acid (13.3-20 mg/L), L-Glutamic acid (14.7-20 mg/L), L-Hydroxy-L-proline (1-20 mg/L), and L-Proline (20-34.5 mg/L) in view of amino acids; cupric sulfate pentahydrate (0.001-0.0025 mg/L), ferrous sulfate heptahydrate (0.4-0.834 mg/L), sodium phosphate dibasic anhydrous (109-800 mg/L) and zinc sulfate heptahydrate (0.1-0.863 mg/L) in view of inorganic salts; D-Biotin (0.0073-0.2 mg/L), P-aminobenzoic acid (PABA, 0.1-1 mg/L), and vitamin B12 (0.005-1.36 mg/L) in view of vitamins; and hypoxanithine (1-4.08 mg/L), L-glutathione reduced (0.1-1 mg/L), linoleic acid (0.01-0.084 mg/L), putrescine+2HCL (0.1-0.161 mg/L), thioctic acid (0.1-0.21 mg/L), and thymindine (0.35-0.73 mg/L) in view of the other components.

The note columns of table 1 with respect to the advanced basic media-mesenchymal stem cell (ABM-M), which is the basic culture medium for mesenchymal stem cells of the present invention, will be described as follows.
(1) The overlapping component of the respective media is selected to have a higher concentration, and if the overlapping component is not contained in the DMEM high glucose, the overlapping component is allowed to be contained in the DMEM high glucose medium through an addition thereof. This corresponds to ① in note columns of table 1.

With respect to this feature, as a result of culturing mesenchymal stem cells derived from adult stem cells of the present disclosure in the ABM-C, which is composed of DMEM and RPMI-1640, of patent document 9 according to the conventional art, morphologic abnormality and adhesiveness loss were caused, and in order to compensate for these, the components relevant to the growth rate and synthesis of cells were supplemented.

As for the amino acids, glycine, L-glutamine, L-isoleucine, L-leucine, L-lysine, L-methionine, L-phenylalaine, L-serine, L-threonine, L-tryptophan, L-tyrosine, and L-valine, which are main amino acids of protein synthesis, were selected to have higher concentrations, thereby promoting the synthesis of proteins and increasing the proliferation of cells.

As for inorganic salts, potassium chloride and sodium chloride, which are relevant to intracellular and extracellular osmotic pressures, were selected to have higher concentrations in order to keep suitable osmotic pressures.

As for the vitamins, ascorbic acid phosphate, choline chloride, D-biotin (allowed to be contained in DMEM high glucose), D-Ca pantothenate, folic acid, myo-Inositol, nicotinamide, riboflavin, thiamine hydrochloride, and vitamin B12 (allowed to be contained in DMEM high glucose), which are antioxidants, were selected to have higher concentrations in order to remove wastes generated due to the high proliferation of cells.

As for the other components, D-glucose anhydrous, which is a main energy source, was selected to have a higher concentration in order to maintain high proliferation, and sodium pyruvate, which is involved in intracellular synthesis, was selected to have a higher concentration. In addition, phenol red sodium salt is not relevant to the proliferation of cells, but the color of phenol red turns from red to yellow by the waste excreted due to the active proliferation of cells, and thus the phenol red sodium salt was selected to have a higher concentration in order to visually measure the amount of the waste excreted due to a high growth rate of cells.

In addition, proline is a main component of collagen, and a main component of extracellular substrate synthesis. Proline was allowed to be contained in the DMEM high glucose in order to increase the synthesis of collagen in mesenchymal stem cells with high proliferation.

In addition, aspartic acid is contained in most proteins, and is connected to the citric acid cycle to serve as an amino group supply source of purine and pyrimidin bases. Therefore, purine and pyrimidin bases are main supply sources of DNA synthesis of cells showing high proliferation, and purine and pyrimidin bases as main supply sources were allowed to be contained in DMEM high glucose.

In addition, L-glutamic acid, which is an important amino acid in the cellular metabolism, was allowed to be contained in the DMEM high glucose in order to achieve active proliferation of cells.
(2) The component contained in only one of the respective media is selected to maintain the concentration thereof *per se,* and if the component is not contained in the DMEM high glucose, the component is allowed to be contained in the DMEM high glucose medium through an addition thereof. This corresponds to ② in note columns of table 1.

The mesenchymal stem cells in the ABM-C, which is composed of DMEM and RPMI-1640 to increase the growth rate, lost cell adhesiveness thereof. In order to compensate for this, the components absent in the DMEM high glucose were allowed to be contained in DMEM high glucose in order to increase the synthesis of intracellular collagen and remove wastes caused due to the increased synthesis.

L-Alanine as an amino acid, which is involved in the immunity and synthesis of cells, was allowed to be contained in order to increase the proliferation of mesenchymal stem cells. L-hydroxy-L-proline as an amino acid, thioctic acid and linoleic acid as other components, and P-aminobenzoic acid (PABA) as vitamin, which are constituents of intracellular collagen, were allowed to be contained in the DMEM high glucose in order to increase the synthesis of intercellular collagen to enhance cell adhesiveness.

Cupric sulfate pentahydrate and zinc sulfate heptahydrate as inorganic salts and glutathione reduced as other component, which are antioxidants, were allowed to be contained in the DMEM high glucose in order to further increase the excretion of various wastes in cells showing a high growth rate to exhibit a high cell growth rate.

Thymidine, putrescine+2HCL, and hypoxanthine as other components, which are involved in DNA synthesis, were allowed to be contained in the DMEM high glucose since DNA synthesis needs to take precedence in order to increase the cell proliferation.
(3) Among the components of the respective media, one component selected from components corresponding to the same supply sources is selected to have a higher concentration if the selected component is an overlapping component, and the selected component was allowed to be contained in the DMEM high glucose through an addition thereof if the selected component is not contained in the DMEM high glucose. This corresponds to ③ in note columns of table 1.

With respect to L-arginine, L-asparagine, L-cysteine/cystine, and L-Histidine, as amino acids; calcium chloride/nitrate, ferric nitrate/ferrous sulfate, and sodium phosphate, as inorganic salts; and pyridoxal hydrochloride/pyridoxine hydrochloride as vitamins, the addition of two or more components thereof corresponding to the same supply source increases the osmotic pressure in the media, badly influencing cells, and thus, of the components corresponding to the same supply source, only one component with a higher concentration was selected.

In addition, of the components of inorganic salts, components with fewer hydrates were selected in order to minimize salts that may be generated at the time of preparation of media.

Exceptionally, sodium phosphate was selected to have a concentration of 142.04 mg/L in order to keep a suitable osmotic pressure since sodium phosphate with a higher concentration may increase the entire osmotic pressure in the medium, resulting in badly influencing cells.

Magnesium chloride hexahydrate and magnesium sulfate anhydrous are supply sources of magnesium, which is a main material of intracellular metabolism. In order to reduce the generation of salts at the time of preparation of media, magnesium sulfate anhydrous was selected, and a higher concentration thereof was selected since magnesium sulfate anhydrous is an overlapping component.

For the reasons above, as for the amino acids, L-arginine monobydrochloride (211 mg/L) is selected between L-arginine free base (RPMI) and L-arginine monobydrochloride (DMEM and F12); L-cystine dihydrochloride (62.6 mg/L) is selected between L-cysteine monobydrochloride monohydrate and L-cystine dihydrochloride; and L-histidine monobydrochloride monohydrate (42 mg/L) is selected between L-histidine and L-histidine monobydrochloride monohydrate, as shown in table 1. As for the inorganic salts, calcium chloride dehydrate (265 mg/L) is selected from calcium chloride dehydrate and calcium nitrate tetrahydrate; ferrous sulfate heptahydrate (0.834 mg/L) is selected between ferric nitrate nonahydrate and ferrous sulfate heptahydrate; magnesium sulfate anhydrous (97.67 mg/L) is selected between magnesium chloride hexahydrate and magnesium sulfate anhydrous; and sodium phosphate dibasic anhydrous (142.04 mg/L) is selected between sodium phosphate dibasic anhydrous and sodium phosphate monobasic anhydrous.

As for the vitamins, pyridoxal hydrochloride (4 mg/L) is selected between pyridoxal hydrochloride and pyridoxine hydrochloride.

As described above, the composition of the basic culture medium for mesenchymal stem cells (advanced basic media-mesenchymal stem cell, ABM-M) for *in vitro* culturing mesenchymal stem cells derived from adult cells, such as bone marrow and fat, starts from the compositions of DMEM high glucose, RPMI-1640, and Ham's F-12, and here, the components of the DMEM high glucose are used as basic components.

That is, since the mesenchymal stem cells are derived from adult stem cells of which the proliferation and synthesis are inactive, the ABM-M of the present invention is a medium in which the components of the DMEM high glucose with high concentrations of amino acids are used as basic components and the concentrations of amino acids, inorganic salts, and vitamins, which are absent or reduced in the DMEM high glucose, are adjusted.

The term mesenchymal stem cells refers to undifferentiated cells which are derived from adult tissues of mammals including humans and have multipotency, and the adult tissues include bone marrow, blood, brain, skin, fat, cord blood, and the like.

The mesenchymal stem cells may be isolated from the adult tissue, such as bone marrow or adipose tissue, through various methods. For example, the mesenchymal stem cells may be isolated using density gradient centrifugation of percoll et.al., (Majumdar MK et al., J. Cell Physiol. 176:57, 1998; Majka SM et al., J. Clin. Invest., 111:71, 2003), or as for the cells isolated by treatment with an enzyme, such as collagenase, all the cells adhering to and growing on the bottom in a culture container may be easily isolated by the method of Luria, et.al., (Luria et al., Transfusion, 11:345, 1971).

The basic culture medium for mesenchymal stem cells of the present invention is for culturing mesenchymal stem cells isolated from adult tissues, such as marrow, blood, brain, skin, fat, and cord blood. In cases of necessity, one or more components may be added thereto, and antibiotic agents and antifungal agents for preventing microbial contamination as well as fetal bovine, equine, or human serum and L-glutamine may be added. Preferably, 10-20% fetal bovine serum and 2-4 mM L-glutamine are added.

In order to verify the growth rate through the basic culture medium for mesenchymal stem cells (ABM-M), the mesenchymal stem cells are cultured in the basic culture medium of the present invention. With respect to cell immunological characteristics of the thus cultured mesenchymal stem cells, the mesenchymal stem cells exhibit cell immunological characteristics that CD166, CD105, CD90, CD44, CD29, CD73, and HLA-ABC positive surface markers are expressed at 80% or more, and particularly, CD44, CD105, CD90, CD73, and CD166 positive surface markers are expressed at 95% or more, and CD31, CD34, CD45, CD80, and HLA-DR negative surface markers are expressed at 5% or less.

In addition, the thus cultured mesenchymal stem cells exhibit spindle-shaped morphology by adhering and proliferating on a plastic culture container, and have differentiation capacity of proliferating in a dedifferentiation state.

In addition, it could be verified that the cultured mesenchymal stem cells are multipotent mesenchymal stem cells that can differentiate into osteoblasts, chondrocytes, and adipocytes.

Furthermore, the present disclosure provides a cell therapeutic agent for treating bone defects, containing mesenchymal stem cells that are cultured in advanced basic media-mesenchymal stem cell (ABM-M) and again cultured in α-MEM containing fetal bovine serum, dexamethasone, β-glycerophosphate, and ascorbic acid, thereby enabling the differentiation into osteoblasts.

Furthermore, the present disclosure provides a cell therapeutic agent for treating ostarthritis, containing mesenchymal stem cells that are cultured in advanced basic media-mesenchymal stem cell (ABM-M) and again cultured in DMEM low glucose containing dexamethasone, ascorbic acid, sodium pyruvate, TGF-β, and BMP-2, thereby enabling the differentiation into chondrocytes.

Furthermore, the present disclosure provides a cell therapeutic agent for forming adipose tissue, containing mesenchymal stem cells that are cultured in advanced basic media-mesenchymal stem cell (ABM-M) and again cultured in α-MEM containing fetal bovine serum, dexamethasone, indomethacin, and insulin, thereby enabling the differentiation into adipocytes.

### Mode for Carrying Out the Invention

Hereinafter, the proliferation capacity of the mesenchymal stem cells in the basic culture medium for mesenchymal stem cells (advanced basic media-mesenchymal stem cell, ABM-M) of the present invention was compared with those in other media, and the verification on cell immunological characteristics of the mesenchymal stem cells cultured in the ABM-M, the verification on differentiation capacity of the mesenchymal stem cells cultured in the ABM-M, and the maintenance of cell karyotype of the mesenchymal stem cells cultured in the ABM-M will be described in detail through examples below.

### [Example 1]

### Comparison of proliferation capacity in basic culture medium for mesenchymal stem cells (ABM-M) of present invention

In order to analyze the proliferation capacity of mesenchymal stem cells derived from adult tissues, such as bone marrow and adipose tissues, in ABM-M, frozen marrow-derived mesenchymal stem cells and adipose-derived mesenchymal stem cells are purchased from Lonza. The bone marrow-derived mesenchymal stem cells were cultured in Poietics MSCGM™ Bullekit, and the adipose-derived mesenchymal stem cells were cultured in Poietics ADSC-GM™ Bullekit, and compared with exclusive media.

### 1-1. Preparation of bone marrow- and adipose tissue-derived mesenchymal stem cells

Bone marrow-derived mesenchymal stem cells purchased from Lonza were promptly thawed in a double boiler at 37°C, placed in 15 mL tube containing 5 mL MSCGM™, and then centrifuged at 300 g for 5 minutes. After the centrifugation, supernatant was removed and the cells were dispensed in 10 mL of new MSCGM to measure cell count and cell viability. The prepared cells were inoculated in T25 flask at 5,000 cells per cm², and cultured while maintaining 37°C and 5% CO₂. The culture liquid was exchanged with 5 mL MSCGM for each culture container every 3-4 days, and when the cells grew to 90% or more of the area of the flask bottom, subculture was conducted. Second, third, and fourth cultures were conducted to prepare cell water for testing.

Adipose tissue-derived mesenchymal stem cells purchased from Lonza were promptly thawed in a double boiler at 37°C, placed in 15 mL tube containing 5 mL ADSC-GM, and then centrifuged at 300 g for 5 minutes. After the centrifugation, supernatant was removed and the cells were dispensed in 10 mL of new ADSC-GM to measure cell count and cell viability. The prepared cells were inoculated in T25 flask at 5,000 cells per cm², and cultured while maintaining 37°C and 5% CO₂. The culture liquid was exchanged with 5 mL ADSC-GM for each culture container every 3-4 days, and when the cells grew to 90% or more of the area of the flask bottom, subculture was conducted. Second, third, and fourth subcultures were conducted to prepare cell water for testing.

### 1-2. Comparison of proliferation of bone marrow-derived mesenchymal stem cells in ABM-M

The proliferation capacity in the ABM-M of the present invention was investigated using the bone marrow-derived mesenchymal stem cells subcultured up to four passages in MSCGM, and here, the MSCGM was used as a control. Since the MSCGM is composed of MSC-BM as a basic medium and additives (including 50 mL growth supplement, 10 mL L-glutamine, and 0.5 mL penicillin-streptomy), additives were also added to ABM-M, and then a comparative test was conducted (Table 2).

**[Table 2]**

| Medium composition for proliferation capacity of ABM-M of present invention and MSCGM | | | | |
|---|---|---|---|---|
| | | Additives (Lonza) | 10% fetal bovine serum | 2mM L-glutamine |
| | MSC-BM+FBS | | ○ | ○ |
| | MSC-BM+ Additives | ○ | | |
| | ABM-M+FBS | | ○ | ○ |
| | ABM-M+ Additives | ○ | | |

After the fourth subculture, the cells were inoculated at 5,000 cells per cm² in T75 flask containing media composition of table 2, and cultured while maintaining 37°C and 5% CO₂. The cells were cultured for 10 days while the culture liquid was exchanged with each media for each culture container every 3-4 days, and then the cell count was measured.

Through FIGS. 1 and 3, with respect to the control groups, the proliferation never occurred in the medium (MSC-BM+FBS) containing MSC-BM supplemented with 10% fetal bovine serum for 10 days, and also hardly occurred in the MSCGM, which is the MSC-BM supplemented with additives.

However, the proliferation was higher in the medium (ABM-M+FBS) containing the ABM-M of the present invention supplemented with 10% fetal bovine serum (FBS), as a test group, rather than MSCGM, and the doubling time was also faster in ABM-M+FBS rather than MSCGM. The higher proliferation and faster doubling time were showed in the medium containing the ABM-M supplemented with additives rather than MSCGM, and thus the composition of the ABM-M was verified to increase the growth rate of bone marrow-derived mesenchymal stem cells.

The bone marrow-derived mesenchymal stem cells did not proliferate in MSCGM, but the bone marrow-derived mesenchymal stem cells proliferated in ABM-M containing 10% fetal bovine serum and 2 mM L-glutamine. This indicates that the proliferation capacity of the mesenchymal stem cells cultured in MSCGM was lost, but the proliferation capacity thereof was maintained in ABM-M.

In order to verify the maintenance of the proliferation capacity in ABM-M, the cells recovered in the fifth subculture were inoculated at 5,000 per cm² in T150 flask, and cultured while maintaining 37°C and 5% CO₂. The cells were cultured for 10 days while the culture liquid was exchanged with ABM-M containing 10% fetal bovine serum and 2 mM L-glutamine for each culture container every 3-4 days, and then the cell count was measured. As a result, 3,357,500 cells were recovered, which showed a growth rate of 447% compared with 750,000 inoculated cells, and thus the proliferation capacity was verified to be maintained. Therefore, it was verified that the ABM-M of the present invention allowed excellent proliferation capacity of bone marrow-derived mesenchymal stem cells.

**[Table 3]**

| Analysis results of proliferation of bone marrow-derived mesenchymal stem cells for each culture media | | | | | |
|---|---|---|---|---|---|
| | | **Number of cells inoculated** | **Number of cells adhering on ist day of culture** | **Number of cells recovered on 10th day of culture** | Doubling time (days) |
| **Control group** | MSC-BM+FBS | 375,000 | 285,000 | 289,335 | 413.16 |
| | MSC-BM+Additives | 375,000 | 378,750 | 457,665 | 32.95 |
| **Test group** | ABM+FBS | 375,000 | 360,000 | 983,500 | 6.21 |
| | ABM+ Additives | 375,000 | 378,750 | 701,000 | 10.13 |

### 1-3. Comparison of proliferation of adipose-derived mesenchymal stem cells in ABM-M

The proliferation capacity in the ABM-M of the present invention was investigated using the adipose-derived mesenchymal stem cells that were subcultured up to four passages in ADSC-GM, and here, ADSC-GM was used as a control. Since ADSC-GM is composed of ADSCC-BM as a basic medium and additives (including 50 mL fetal bovine serum, 5 mL L-glutamine, 0.5 mL gentamicin-amphotercin), 10% fetal bovine serum and 2 mM L-glutamine were also added to ABM-M of the present invention, and then a comparative test was conducted. After the fourth subculture, the cells were inoculated at 5,000 cells per cm² in T75 flask containing each media, and cultured while maintaining 37°C and 5% CO₂. The cells were cultured for 7 days while the culture liquid was exchanged with each media for each culture container every 3-4 days, and then the cell count was measured. In addition, the recovered cells were subcultured, and then inoculated at 5,000 cells per cm² in T175 flask containing each media, and cultured while maintaining 37°C and 5% CO₂. The cells were cultured for 7 days while the culture liquid was exchanged with each media for each culture container every 3-4 days, and then the cell count was measured.

Through FIGS. 2 and 4 and table 5, the doubling time in the fifth and sixth subculture was faster in ABM-M supplemented with 10% fetal bovine serum (ABM-M+FBS) as a test group rather than ADSC-GM as a control, and the recovered cells are more in ABM-M+FBS rather than ADSC-GM. Therefore, it was verified that the proliferation capacity of adipose-derived mesenchymal stem cells was increased by ABM-M of the present invention (table 4).

**[Table 4]**

| Analysis results of proliferation of adipose-derived mesenchymal stem cells for each culture media | | | | |
|---|---|---|---|---|
| | ADSC-GM | | ABM-M+FBS | |
| | Passage 5 | Passage 6 | Passage 5 | Passage 6 |
| **Number of cells inoculated** | | 875,000 | 375,000 | 875,000 |
| **Number of cells adhering on 1St day of culture** | 373,125 | 761,250 | 390,000 | 843,750 |
| **Number of cells recovered on 7th day of Culture** | 935,500 | 2,219,000 | 1,161,000 | 4,443,500 |
| **Doubling time** (days) | 4.5 | 3.9 | 3.8 | 2.5 |

### [Example 2]

### Analysis on immunological characteristics of mesenchymal stem cells cultured in ABM-M of present invention

The cells cultured in culture media of 1-2 and 1-3 of example 1 were isolated by the treatment with TrypLE express, and centrifuged at 400 g for 5 minutes, thereby obtaining cells. Then, the cells were washed two times with FASC solution, and the washed cells were dispensed at 5 10⁵, and reacted with anti-CD166, CD105, CD90, CD44, CD29, CD73, HLA-ABC, CD31, CD34, CD45, CD80, and HLA-DR antibodies for 20 minutes, followed by washing, and then allowed to float in FACS solution, followed by flow cytometry analysis. The cell surface was analyzed to identify the phenotype of the mesenchymal stem cells.

As a result, like the mesenchymal stem cells cultured in MSC-GM and ADSC-GM as control groups, the cells cultured in ABM-M containing 10% fetal bovine serum and 2 mM L-glutamine showed positive expression for CD166, CD105, CD90, CD44, CD29, CD73, and HLA-ABC and negative expression for CD14, CD31, CD34, CD45, CD80, and HLA-DR, and thus it was verified that the cultured cells have characteristics of mesenchymal stem cells (table 5).

**[Table 5]**

| Immunological characteristics of mesenchymal stem cells cultured in ABM-M | | | | |
|---|---|---|---|---|
| | **Bone marrox-derived mesenchymal stem cells** | | **Adloose-derived mesenchymal stem cells** | |
| | MSC-GM | ABM-M | ADSC-GM | ABM-M |
| CD29 | 76.6% | 83.2% | 88.7% | 97.7% |
| CD44 | 95.2% | 98.0% | 99.9% | 100% |
| CD73 | N/A | N/A | 98.7% | 98.5% |
| CD90 | 91.86% | 93.25% | 97.4% | 98.0% |
| CD105 | 99.6% | 98.6% | 99.9% | 100% |
| CD166 | 99.5% | 99.5% | 100% | 99.9% |
| HLA-ABC | 76.7% | 82.2% | 83.8% | 92.1% |
| CD80 | 0.0% | 0.1% | 0.2% | 0.1% |
| CD45 | 3.9% | 9.12% | 0.2% | 3.0% |
| CD34 | 0.2% | 0.1% | 5.2% | 14.6% |
| CD31 | 0.1% | 1.9% | 0.1% | 0,6% |
| CD14 | 0.2% | 0.09% | 0.8% | 3.0% |
| HLA-DR | 0.1% | 0.0% | 0.1% | 0.1% |

### [Example 3]

### Analysis on differentiation capacity of mesenchymal stem cells cultured in ABM-M

The cells cultured in culture media of 1-2 and 1-3 of example 1 were isolated by the treatment with triplex, and centrifuged at 400 g for 5 minutes, thereby obtaining cells. Then, the multipotency of the obtained cells was verified under *in vitro* conditions.

In order to verify multipotency of the cultured mesenchymal stem cells, differentiation was induced by the method of Schallmoser K, et al., (Schallmoser K. et al., Tissue Eng Par C Method 14:185, 2008) as follows.

As for differentiation into osteoblasts, after the mesenchymal stem cells early-cultured in ABM-M were again cultured and differentiated in α-MEM containing 10% fetal bovine serum, 10 mM β-glycerol phosphate, 50 uM ascorbic acid, and 10⁻⁷ M dexamethasone for 2-3 weeks, the expression of alkaline phosphatase was assayed by ALPase staining, and the accumulation of calcium was confirmed by von Kossa staining, as shown in FIG. 8.

As for differentiation into adipocytes, after the mesenchymal stem cells early-cultured in ABM-M were again cultured and differentiated in α-MEM containing 10% fetal bovine serum, 10⁻⁷ M dexamethasone, 100 uM indomethacin, and 10 ug/mL insulin for 2 weeks, the adipose droplets accumulated in the cells were confirmed through oil Red-O staining.

In order to induce the differentiation into chondrocytes, about 5 x 10⁵ of mesenchymal stem cells early cultured in ABM-M were centrifuged at 300 g for 5 minutes to make a cell mass, and then again cultured and differentiated in the DMEM low glucose containing 10⁻⁷ M dexamethasone, 50 uM ascorbic acid, 1 nM sodium pyruvate, 10 ng/mL TGF-β, and 100 ng/mL BMP-2 for 3 weeks, as shown in FIG. 10. The differentiation-induced chondrocytes were made to obtain 5-um serial sections through a paraffin embedding procedure, and the differentiation capacity into chondrocytes was confirmed through H/E staining, safranin O staining, alcian blue staining, sirius red staining, COMP staining, collagen type II and I staining.

After the bone marrow- and adipose-derived mesenchymal stem cells cultured in ABM-M of the present invention were subcultured by two passages, the multi-differentiation thereof was induced, and comparison with a control group without multi-differentiation was conducted. As a result of conducting ALP staining and calcium deposit after the induction into osteoblasts, the expression of alkaline phosphatase and the deposit of calcium were confirmed unlike in the control group without induced differentiation. In addition, as a result of conducting oil Red-O staining after the induction into adipocytes, the differentiation into adipocytes was confirmed, unlike in the control group without induced differentiation. Through safranin O staining, alcian blue staining, Sirius red staining, COMP staining, and collagen type II and I staining, the expression of glycosaminoglycan (GAG) and proteoglycan, collagen type I was confirmed in the chondrocytes differentiated from the mesenchymal stem cell mass, similar to normal cartilage.

### [Example 4]

### Analysis on karyotype of mesenchymal stem cells cultured in ABM-M

Like 1-2 and 1-3 of example 1, mesenchymal stem cells are isolated from adult tissues, such as bone marrow and adipose tissue derived different objects, and subcultured ten passages in ABM-M containing 10% fetal bovine serum and 2 mM L-glutamine, and then the karyotype of the cultured mesenchymal stem cells was analyzed. Five of 20 metaphase cells were assayed using GTG banding technique, and the karyotypes thereof were written according to International System for Cytogenetic Nomenclature (2009). From all the observed five metaphase cells, FIG. 11 shows normal 46, XY karyotype, and FIG. 12 shows normal 46, XX karyotype.

As described above, the cell characteristics and differentiation capacity of mesenchymal stem cells are maintained in the basic culture medium of mesenchymal stem cells (ABM-M) of the present invention compared with conventional mesenchymal stem cell culture media, and the ABM-M of the present invention is used as media for excellent cell growth and proliferation to mass-culture mesenchymal stem cells, thereby obtaining pure mesenchymal stem cells.

## Claims

1. An advanced basic culture medium for mesenchymal stem cells comprising the components and their respective amounts in mg/L as given in the following table:
| component | amount [mg/l] |
|---|---|
| Glycine | 30 |
| L-alanine | 9 |
| L-arginine monohydrochloride | 211 |
| L-asparagine anhydrous | 50 |
| L-aspartic acid | 20 |
| L-cystine dihydrochloride | 62.6 |
| L-glutamic acid | 20 |
| L-glutamine | 584 |
| L-histidine monohydrochloride | 42 |
| L-hydroxy-L-proline | 20 |
| L-isoleucine | 105 |
| L-leucine | 105 |
| L-lysine monohydrochloride | 146 |
| L-methionine | 30 |
| L-phenylalanine | 66 |
| L-proline | 34.5 |
| L-serine | 42 |
| L-threonine | 95 |
| L-tryptophan | 16 |
| L-tyrosine disodium salt dihydrate | 103.79 |
| L-valine | 94 |
| Calcium chloride dihydrate | 265 |
| Cupric sulfate pentahydrate | 0.0025 |
| Ferrous sulfate heptahydrate | 0.834 |
| Magnesium sulfate anhydrous | 97.67 |
| Potassium chloride | 400 |
| Sodium chloride | 6400 |
| Sodium phosphate dibasic anhydrous | 142.04 |
| Zinc sulfate heptahydrate | 0.863 |
| Ascorbic acid phosphate | 50 |
| Choline chloride | 13.96 |
| D-biotin | 0.2 |
| D-Ca panthothenate | 4 |
| Folic acid | 4 |
| Myo-inositol | 35 |
| Nicotinamide (nicotinic acid amide) | 4 |
| p-aminobenzoic acid (PABA) | 1 |
| Pyridoxal hydrochloride | 4 |
| Riboflavin | 0.4 |
| Thiamine hydrochloride | 4 |
| Vitamine B12 | 1.36 |
| D-glucose anhydrous | 4500 |
| hypoxanthine | 4.08 |
| L-glutathione reduced | 1 |
| Linoleic acid | 0.084 |
| Phenol red sodium salt | 15.9 |
| Putrescine+2HCl | 0.161 |
| Sodium pyruvate | 110 |
| Thioctic acid | 0.21 |
| thymidine | 0.73 |

2. The advanced basic culture medium for mesenchymal stem cells according to claim 1, in which mesenchymal stem cells exhibit cell immunological characteristics that CD166, CD105, CD90, CD44, CD29, CD73, and HLA-ABC positive surface markers are expressed at 80% or more, especially CD44, CD105, CD90, CD73, and CD166 positive surface markers are expressed at 95% or more, and CD31, CD34, CD45, CD80, and HLA-DR negative surface markers are expressed at 5% or less.

## Patentansprüche

1. Fortgeschrittenes basisches Kulturmedium für mesenchymale Stammzellen, das die Komponente und ihre jeweiligen Mengen in mg/l umfasst, wie sie in der folgenden Tabelle angegeben sind:
| Komponente | Menge [mg/l] |
|---|---|
| Glycin | 30 |
| L-Alanin | 9 |
| L-Arginin-Monohydrochlorid | 211 |
| L-Asparagin wasserfrei | 50 |
| L-Asparaginsäure | 20 |
| L-Cystin-Dihydrochlorid | 62,6 |
| L-Glutaminsäure | 20 |
| L-Glutamin | 584 |
| L-Histidin-Monohydrochlorid | 42 |
| L-Hydroxy-L-prolin | 20 |
| L-Isoleucin | 105 |
| L-Leucin | 105 |
| L-Lysin-Monohydrochlorid | 146 |
| L-Methionin | 30 |
| L-Phenylalanin | 66 |
| L-Prolin | 34,5 |
| L-Serin | 42 |
| L-Threonin | 95 |
| L-Tryptophan | 16 |
| L-Tyrosin-Dinatriumsalz-Dihydrat | 103,79 |
| L-Valin | 94 |
| Calciumchlorid-Dihydrat | 265 |
| Kupfer(II)sulfat-Pentahydrat | 0,0025 |
| Eisen(II)sulfat-Heptahydrat | 0,834 |
| Magnesiumsulfat wasserfrei | 97,67 |
| Kaliumchlorid | 400 |
| Natriumchlorid | 6400 |
| Natriumdihydrogenphosphat wasserfrei | 142,04 |
| Zinksulfat-Heptahydrat | 0,863 |
| Ascorbinsäurephosphat | 50 |
| Cholinchlorid | 13,96 |
| D-Biotin | 0,2 |
| D-Ca-Pantothenat | 4 |
| Folsäure | 4 |
| Myo-Inosit | 35 |
| Nicotinamid (Nicotinsäureamid) | 4 |
| p-Aminobenzoesäure (PABA) | 1 |
| Pyridoxal-Hydrochlorid | 4 |
| Riboflavin | 0,4 |
| Thiamin-Hydrochlorid | 4 |
| Vitamin B12 | 1,36 |
| D-Glucose wasserfrei | 4500 |
| Hypoxanthin | 4,08 |
| L-Glutathion reduziert | 1 |
| Linoleinsäure | 0,084 |
| Phenolrot-Natriumsalz | 15,9 |
| Putrescin+2HCl | 0,161 |
| Natriumpyruvat | 110 |
| Thioctsäure | 0,21 |
| Thymidin | 0,73 |

2. Fortgeschrittenes basisches Kulturmedium für mesenchymale Stammzellen gemäß Anspruch 1, wobei mesenchymale Stammzellen die immunologischen Zellmerkmale aufweisen, dass die positiven Oberflächenmarker CD166, CD105, CD90, CD44, CD29, CD73 und HLA-ABC zu 80% oder mehr exprimiert werden, insbesondere die positiven Oberflächenmarker CD44, CD105, CD90, CD73 und CD166 zu 95% oder mehr exprimiert werden, und die negativen Oberflächenmarker CD31, CD34, CD45, CD80 und HLA-DR zu 5% oder weniger exprimiert werden.

## Revendications

1. Milieu de culture basique avancé pour des cellules souches mésenchymales, comprenant les composants et leurs quantités respectives en mg/l comme indiqués dans le tableau suivant :
| Composant | Quantité [mg/l] |
|---|---|
| glycine | 30 |
| L-alanine | 9 |
| monohydrochlorure de L-arginine | 211 |
| L-asparagine anhydre | 50 |
| acide L-aspartique | 20 |
| dihydrochlorure de L-cystine | 62,6 |
| acide L-glutamique | 20 |
| L-glutamine | 584 |
| monohydrochlorure de L-histidine | 42 |
| L-hydroxy-L-proline | 20 |
| L-isoleucine | 105 |
| L-leucine | 105 |
| monohydrochlorure de L-lysine | 146 |
| L-méthionine | 30 |
| L-phénylalanine | 66 |
| L-proline | 34,5 |
| L-sérine | 42 |
| L-thréonine | 95 |
| L-tryptophane | 16 |
| L-tyrosine disodique dihydraté | 103,79 |
| L-valine | 94 |
| chlorure de calcium dihydraté | 265 |
| sulfate de cuivre pentahydraté | 0,0025 |
| sulfate ferreux heptahydraté | 0,834 |
| sulfate de magnésium anhydre | 97,67 |
| chlorure de potassium | 400 |
| chlorure de sodium | 6400 |
| dihydrogénophosphate de sodium anhydre | 142,04 |
| sulfate de zinc heptahydraté | 0,863 |
| phosphate d'acide ascorbique | 50 |
| chlorure de choline | 13,96 |
| D-biotine | 0,2 |
| D-pantothénate de Ca | 4 |
| acide folique | 4 |
| myo-inositol | 35 |
| nicotinamide | 4 |
| acide p-aminobenzoïque (PABA) | 1 |
| hydrochlorure de pyridoxal | 4 |
| riboflavine | 0,4 |
| hydrochlorure de thiamine | 4 |
| vitamine B12 | 1,36 |
| D-glucose anhydre | 4500 |
| hypoxanthine | 4,08 |
| L-glutathione réduit | 1 |
| acide linoléique | 0,084 |
| sel sodique du rouge de phénol | 15,9 |
| putrescine+2HCl | 0,161 |
| pyruvate de sodium | 110 |
| acide thioctique | 0,21 |
| thymidine | 0,73 |

2. Milieu de culture basique avancé pour des cellules souches mésenchymales selon la revendication 1, dans lequel des cellules souches mésenchymales présentent les caractéristiques immunologiques de cellule d'exprimer les marqueurs de surface positifs CD166, CD105, CD90, CD44, CD29, CD73 et HLA-ABC à 80 % ou plus, notamment d'exprimer les marqueurs de surface positifs CD44, CD105, CD90, CD73 et CD166 à 95 % ou plus, et d'exprimer les marqueurs de surface négatifs CD31, CD34, CD45, CD80 et HLA-DR à 5 % ou moins.
